# FASCICULE DE BREVET EUROPEEN

(11) **EP 0 708 759 B1**
(45) Date de publication et mention de la délivrance du brevet: **02.08.2000**
(21) Numéro de dépôt: 95911481.0
(22) Date de dépôt: 28.03.1995
(51) Int. Cl.: C07D 241/12, C07D 241/42

(54) **PROCEDE POUR LA PREPARATION DE PYRAZINES SUBSTITUEES**
VERFAHREN ZU HERSTELLUNG VON SUBSTITUIERTEN PYRAZINEN
METHOD FOR PREPARING SUBSTITUTED PYRAZINES

(30) Priorité: 10.05.1994 CH 144894
(43) Date de publication de la demande: 01.05.1996
(73) Titulaire: FIRMENICH SA, 1211 Genève 8 (CH)
(72) Inventeur: WHITEHEAD, Ian, Michael, CH-1207 Genève (CH)
(74) Mandataire: Salvaterra-Garcia, Maria de Lurdes
(86) Numéro de dépôt international: IB9500212
(87) Numéro de publication internationale: WO9530660

(56) Documents cités:
- EP-A- 0 505 891
- EP-A- 0 590 296
- CHEMICAL ABSTRACTS, vol. 88, no. 15, 1978, Columbus, Ohio, US; abstract no. 105411e, page 582 ;colonne 2 ; & JP,A,7 797 983 (TAKASAGO PERFUMERY) 17 Août 1977

## Description

### Domaine technique

La présente invention a trait au domaine de la synthèse organique et, plus particulièrement, à celui de la préparation de pyrazines substituées.

Les pyrazines substituées, et en particulier les alkylpyrazines, sont des ingrédients aromatisants très importants, pour la plupart d'origine naturelle. Elles sont notamment présentes dans bon nombre de produits fermentés ou soumis à des traitements thermiques.

D'autre part, ces alkylpyrazines constituent également des produits de départ pour la préparation d'autres dérivés pyraziniques et jouent donc un rôle important en synthèse organique.

### Art antérieur

On connaît déjà des procédés de préparation d'alkylpyrazines, particulièrement adaptés à la synthèse de produits naturels. Par exemple, G. P. Rizzi, dans J. Agr. Food Chem. 36, 349 (1988), décrit un procédé pour la préparation d'alkylpyrazines par réaction d'acyloines et d'acétate d'ammonium, dans des conditions de réaction dites "douces". Il s'agit d'un procédé exigeant une procédure compliquée, avec un temps de réaction long. D'autre part, dans la demande de brevet européen n° 505 891, on décrit un procédé faisant usage de la même réaction, mais dans lequel cette dernière est conduite à reflux dans l'eau, le cas échéant avec sublimation simultanée du mélange de réaction.

Ces deux procédés permettent d'obtenir des alkylpyrazines symétriques mais ne fournissent pas une solution satisfaisante au problème posé par la préparation de pyrazines substituées asymétriques, que l'on sait bien plus ardue.

La demande européenne EP-A-590 296 décrit un procédé pour la préparation de triaikylpyrazines asymmétriques. Ledit procédé comprend la réaction, dans un milieu aqueux, d'une 1-hydroxy-2-cétone avec un compose d'ammonium et un aldéhyde, sous des conditions de réaction bien précises, à savoir qu'on ajoute goutte à goutte la 1-hydroxy-2-cétone et l'aldéhyde à la solution contenant le composé d'ammonium.

Bien que ce procédé permette de synthétiser des pyrazines asymmétriques, il est restreint aux triaikylpyrazines et ne fournit pas de solution au problème posé pour la préparation de pyrazines asymmétriques tétrasubstituées ou porteur d'autres substituants que des groupes alkyles.

### Exposé de l'invention

Le but de la présente invention est justement d'apporter une solution nouvelle à ce problème, en réalisant un procédé dont l'application est très générale, lequel permet d'obtenir des pyrazines substituées, d'origine naturelle ou synthétique, et en particulier des alkylpyrazines asymétriques. Ainsi, l'invention concerne un procédé pour la préparation d'une pyrazine de formule 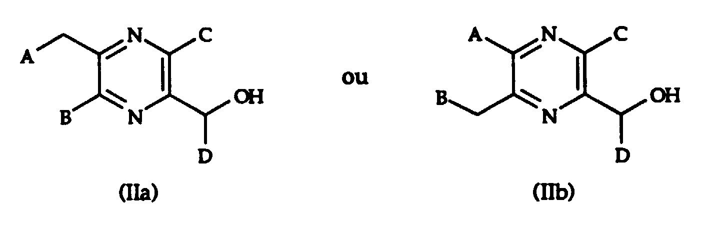 dans laquelle A = B = C = D = R¹ = R², ou dans laquelle A = C = R¹, B = D = R², et R¹ ≠ R², ou A = D = R¹, B = C = R² et R¹ ≠ R², les symboles R¹ et R² représentant chacun un atome d'hydrogène, un radical hydrocarbure saturé ou insaturé, linéaire ou ramifié, ayant de 1 à 6 atomes de carbone ou un groupe -CH₂OH;
ou respectivement de formule 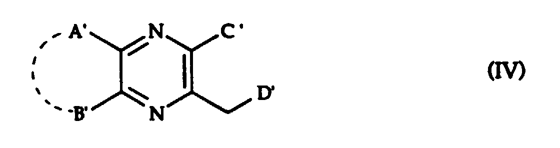 dans laquelle A' = R³, B' = R⁴ ou vice versa et C' = R¹, D' = R², R¹ et R² étant identiques ou différents et ayant le sens indiqué ci-dessus et R³ et R⁴ étant identiques ou différents et représentant chacun un radical hydrocarbure de C₁ à C₆, saturé ou insaturé, linéaire ou ramifié, ou un radical cyclique saturé ou insaturé, ayant 5 ou 6 atomes de carbone dans le cycle, ou étant identiques et représentant un groupe CH ou CH₂ faisant partie d'un cycle tel que représenté par la ligne pointillée, contenant 5 ou 6 atomes de carbone, le procédé étant caractérisé en ce qu'on fait réagir dans un solvant organique inerte un composé de formule 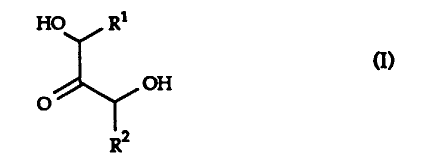 dans laquelle R¹ et R² sont identiques ou différents et ont le sens indiqué ci-dessus, avec un sel d'ammonium, ou respectivement avec un sel d'ammonium et une acyloine de formule 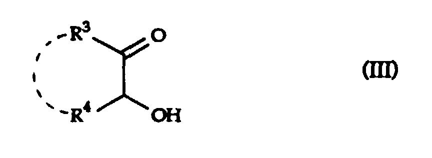 dans laquelle R³ et R⁴ ont le sens indiqué à la formule (IV), et, le cas échéant, on sépare ladite pyrazine du produit de la réaction.

Le procédé de l'invention repose sur le principe de l'utilisation de la dihydroxycétone de formule (I) en tant que substrat réagissant avec le sel d'ammonium. Sans vouloir préjuger du mécanisme de la réaction, on peut admettre que cette di-hydroxycétone réagisse avec elle-même ou avec l'acyloine de formule (III) pour former des (di)-hydroxyméthyl-dihydropyrazines, lesquelles se déshydratent spontanément pour fournir les pyrazines substituées désirées. Ainsi, si l'on prend le cas particulier de la 1,3-dihydroxy-2-propanone, sa réaction avec elle-même fournit l'hydroxypyrazine représentée au schéma suivant:

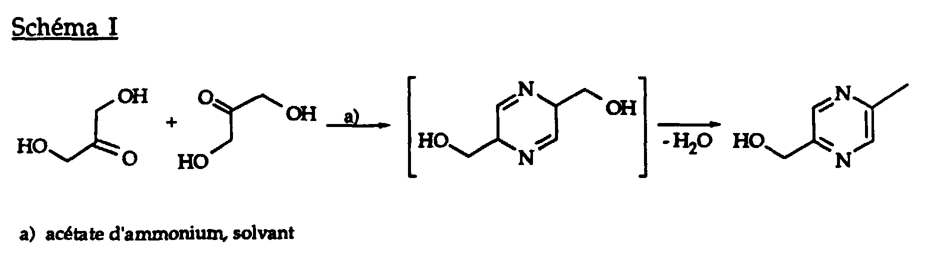

Par ailleurs, on peut observer également la formation de 6-méthyl-2-pyrazineméthanol, toutefois en quantité plus faible que le produit principal susmentionné.

Lorsque l'on fait réagir la 1,3-dihydroxy-2-propanone avec une acyloine de formule (III), on obtiendra en outre des pyrazines substituées telles que représentées dans le schéma suivant: 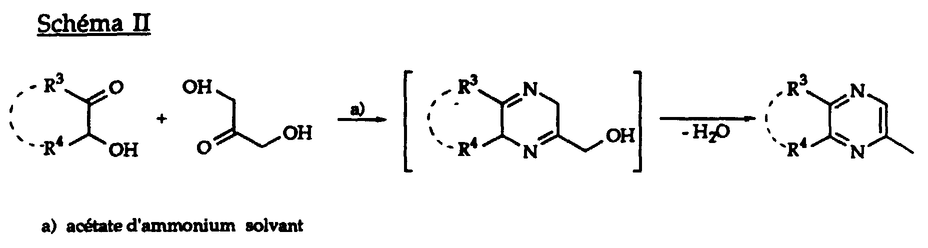 R³ et R⁴ sont définis comme à la formule (III)

De nouveau, lorsque l'acyloine (III) possède des substituants R³ et R⁴ non identiques, on pourra observer aussi la formation de pyrazines isomères de celle représentée, probablement en résultat de la tautomérie cétoénolique du composé (I). Quoiqu'un tel équilibre tautomérique soit aussi possible pour l'acyloine (III), on n'a pas observé la formation de pyrazines qui pourraient en résulter.

Dans les deux cas, les pyrazines individuelles composant le produit de la réaction peuvent être isolées par des méthodes courantes telles que distillation fractionnée, chromatographie, ou autres méthodes de séparation courantes.

Il convient de noter que les conditions de réaction utilisées sont également propices à la formation des pyrazines symétriques qui résultent de la réaction de l'acyloine (III) avec le sel d'ammonium, de façon analogue à celle décrite dans les références de l'art antérieur citées auparavant.

Ainsi, et selon la nature des réactifs de formule (I), ou de formules (I) et (III), le produit de la réaction peut être un mélange plus ou moins complexe de pyrazines asymétriques de formule (II), pouvant également contenir une ou plusieurs pyrazines asymétriques de formule (IV), lesquelles sont ensuite isolées du mélange réactionnel.

Par "sel d'ammonium", on veut dire ici un sel d'ammonium dérivé d'un acide carboxylique, sulfamique ou minéral, de préférence choisi dans le groupe constitué par l'acétate d'ammonium, le citrate d'ammonium, le tartrate d'ammonium, le formiate d'ammonium, l'oxalate d'ammonium, le succinate d'ammonium, le lactate d'ammonium, le sulphamate d'ammonium, le chlorure d'ammonium, le sulphate d'ammonium et le phosphate (II) ou (III) d'ammonium. Selon un mode d'exécution préférentiel, on utilise l'acétate ou le formiate d'ammonium.

La réaction a lieu dans un solvant organique inerte. Dans cette capacité, on peut utiliser un solvant quelconque, qui soit inerte dans les conditions de la réaction. A titre de solvants préférés, on peut citer le méthanol, l'éthanol, le propanol, l'isopropanol, le toluène, l'acétate d'éthyle ou encore des mélanges de deux ou plusieurs de ces solvants. Nous avons constaté également que les meilleurs rendements en produit final étaient obtenus lorsque le solvant était anhydre. Selon un mode d'exécution préférentiel, on utilisera de l'éthanol anhydre.

Les produits de départ dans le procédé de l'invention sont des produits commerciaux ou qui peuvent être facilement préparés à partir de produits commercialisés. Lorsqu'ils sont disponibles sur le marché sous forme de solutions aqueuses, on élimine l'eau avant leur utilisation. D'autre part, lorsque l'on désire préparer des pyrazines destinées à être utilisées dans des arômes naturels, on choisira des produits de départ de qualité appropriée et dont la nature obéit aux normes législatives en vigueur.

Les proportions relatives des produits de départ (I) et (III) peuvent varier dans une gamme de valeurs assez étendue sans que le rendement global de la réaction en pyrazines soit trop influencé. Cependant, le fait que chacun de ces produits de départ puisse réagir avec lui-même a une influence sur les rendements individuels de chacune des pyrazines formées, lesquels rendements peuvent être modifiés en changeant le rapport molaire entre les composés (I) et (III) (voir tableaux présentés plus loin).

Ainsi, bien que l'on puisse utiliser ces composés dans un rapport composé (I) / composé (III) allant typiquement de 1:1 jusqu'à 1:3, nous avons constaté que lorsque l'on utilisait l'acyloine en une quantité supérieure au rapport stoichiométrique, disons en un excès de 2 à 1, on observait une augmentation dans le rendement en pyrazine asymétrique résultant de la réaction des composés (I) et (III), au détriment du rendement en pyrazine symétrique résultant de la réaction d'addition (III) : (III). En même temps, on a aussi observé une augmentation en hydroxypyrazine résultant de l'addition (I) : (I). On pourra donc choisir le rapport relatif des composés (I) et (III) en fonction du prix des produits de départ et de la nature de la pyrazine finale que l'on désire obtenir en rendement plus élevé.

La réaction peut s'effectuer dans une gamme de températures étendue, variant entre la température ambiante et la température de reflux du solvant utilisé. Selon un mode d'exécution préférentiel, on effectuera cette réaction à la température de reflux du solvant. D'autre part, bien que l'on puisse ajouter une solution du sel d'ammonium au mélange de composés (I), ou (I) et (III), il a été constaté que de meilleurs rendements étaient obtenus lorsque la solution d'acyloine (III) et dihydroxycétone (I), ou uniquement de cette dernière, dans le solvant choisi était ajoutée lentement au sel d'ammonium, à la température de reflux du solvant.

L'invention sera maintenant décrite de façon plus détaillée à l'aide des exemples suivants dans lesquels les températures sont indiquées en degrés centigrades et les abréviations ont le sens usuel dans l'art.

### Manières de réaliser l'invention

### Exemple 1

### Utilisation de la 1,3-dihydroxy-2-propanone en tant que substrat réactionnel

### Méthode générale

On a dissous la 1,3-dihydroxy-2-propanone et, le cas échéant, l'acyloine de formule (III), dans de l'éthanol anhydre en chauffant le mélange à reflux (80°). On a laissé refroidir à température ambiante et placé la solution dans une ampoule d'introduction installée sur un ballon à 3 cols de 500 ml contenant de l'acétate d'ammonium solide (ou un autre sel d'ammonium). On a équipé le ballon d'un réfrigérant à l'eau et d'un agitateur et chauffé à reflux (80°). La solution de 1,3-dihydroxy-2-propanone a ensuite été ajoutée goutte à goutte, pendant 20 min., et le mélange a été maintenu à reflux pendant 2 à 3h. On a laissé refroidir à température ambiante et ajusté le pH à 1 à l'aide de HCl. L'acide acétique et la phase neutre ont été extraits du mélange avec trois volumes d'éther éthylique. Les phases organiques combinées ont été lavées à nouveau avec du HCl 5M et on a ajouté ce dernier à la phase aqueuse. Le pH du mélange a été ajusté à 11 à l'aide de pastilles de NaOH, en maintenant la température à une valeur basse à l'aide d'un bain de glace. On a extrait à nouveau à l'éther (3 fois) et séché les deux extraits organiques sur MgSO₄ anhydre, filtré et évaporé le solvant sous vide.
La fraction basique ainsi obtenue a été distillée sur résidu dans un appareil au four à boules et le produit obtenu analysé par chromatographie en phase gazeuse.

### A. Préparation de 5(6)-méthyl-2-pyrazineméthanol

Obtenu par réaction de 46 g (∼ 0,5 mole) de 1,3-dihydroxy-2-propanone (origine: Merck, pur à 99%), dans 60 g d'éthanol, et de 57,8 g (0,75 mole) d'acétate d'ammonium solide, selon le procédé décrit ci-dessus. La fraction acide/neutre (10,18 g) a été séparée comme décrit et la fraction basique (5,52 g) distillée sur résidu à 75° et 8 Pa pour fournir 4,6 g d'une huile jaune pâle dont l'analyse chromatographique présentait un pic unique (pureté : 98,5%; rendement total en pyrazine : 14,3%).
L'analyse GC-MS, RMN(¹H) et RMN(¹³C) de ce produit a montré qu'il s'agissait d'un mélange de 5-méthyl-2-pyrazineméthanol et de 6-méthyl-2-pyrazineméthanol (isomère majeur : isomère mineur 3:2).
- RMN(¹H,360 MHz):: isomère majeur : 2,57(s,3H); 4,78(s,2H); 8,39(2,1H); 8,54(s,1H) δ ppm
isomère mineur : 2,57(s,3H); 4,78(s,2H); 8,37(2,1H); 8,46(s,1H) δ ppm
- RMN (¹³C):: isomère majeur : 152,7(s); 152,3(s); 143,3(d); 141,8(d); 62,9(t); 21,1(q) δ ppm
isomère mineur : 154,8(s); 152,9(s); 142,9(d); 139,5(d); 63,0(t) ; 21,3(q) δ ppm
- SM :: 124(M⁺,91) ; m/e: 123(59), 95(100), 66(19), 55(27), 42(32), 39(44)

### B. Préparation de 2,3,5-triméthylpyrazine

Obtenue selon la méthode générale décrite, par réaction de 1,3-dihydroxy-2-propanone (22,5 g, 0,25 mole) et acétoine (3-hydroxy-2-butanone; 29,7 g, 0,25 mole) dans 40 ml d'éthanol, et de 57,8 g (0,75 mole) d'acétate d'ammonium solide.
La fraction basique a été distillée sur résidu à 60-65° et 10x10² Pa pour fournir une huile jaune pâle (13,91 g) et 5,05 g de résidu.
L'analyse chromatographique de ce produit a montré que la fraction distillée contenait 81% de 2,3,5-triméthylpyrazine (rend.: 36,8%) et 17% de tétraméthylpyrazine (rend.: 13,9%), alors que le résidu contenait 5,8% de triméthylpyrazine, 13,9% de tétraméthylpyrazine et 40,5% de mélange de 5(6)-méthyl-pyrazineméthanol (rend. : 13,2%).

La 2,3,5-triméthylpyrazine a été séparée d'une fraction distillée, obtenue de façon identique mais à échelle molaire (63,0 g), par distillation fractionnée sur colonne de type Fischer à 64x10² Pa. Les résultats suivants ont été obtenus :

| Fraction | Point d'ébullition (°C) | Poids (g) | 2,3,5-Triméthylpyrazine (%) |
|---|---|---|---|
| 1 | 55-66 | 2,30 | 0,3 |
| 2 | 66-65 | 5,66 | 5,1 |
| 3 | 65-69 | 1,38 | 5,2 |
| 4 | 69-87 | 2,33 | 45,3 |
| 5 | 87-88 | 16,96 | 98,9 |
| 6 | 85-87 | 8,04 | 99,2 |
| 7 | 87-88 | 11,99 | 97,2 |
| 8 | 88-88 | 1,07 | 65,8 |
| Résidu | | 6,99 | |
| Total | | 56,72 | |

On a combiné les fractions 5 à 7 (poids total 39,99 g) représentant un rendement en produit isolé de 32,8%. Le spectre RMN de ce produit a confirmé qu'il s'agissait de 2,3,5-triméthylpyrazine pure.
Le tableau I suivant indique les rendements obtenus lorsque les conditions de la réaction ont été variées.

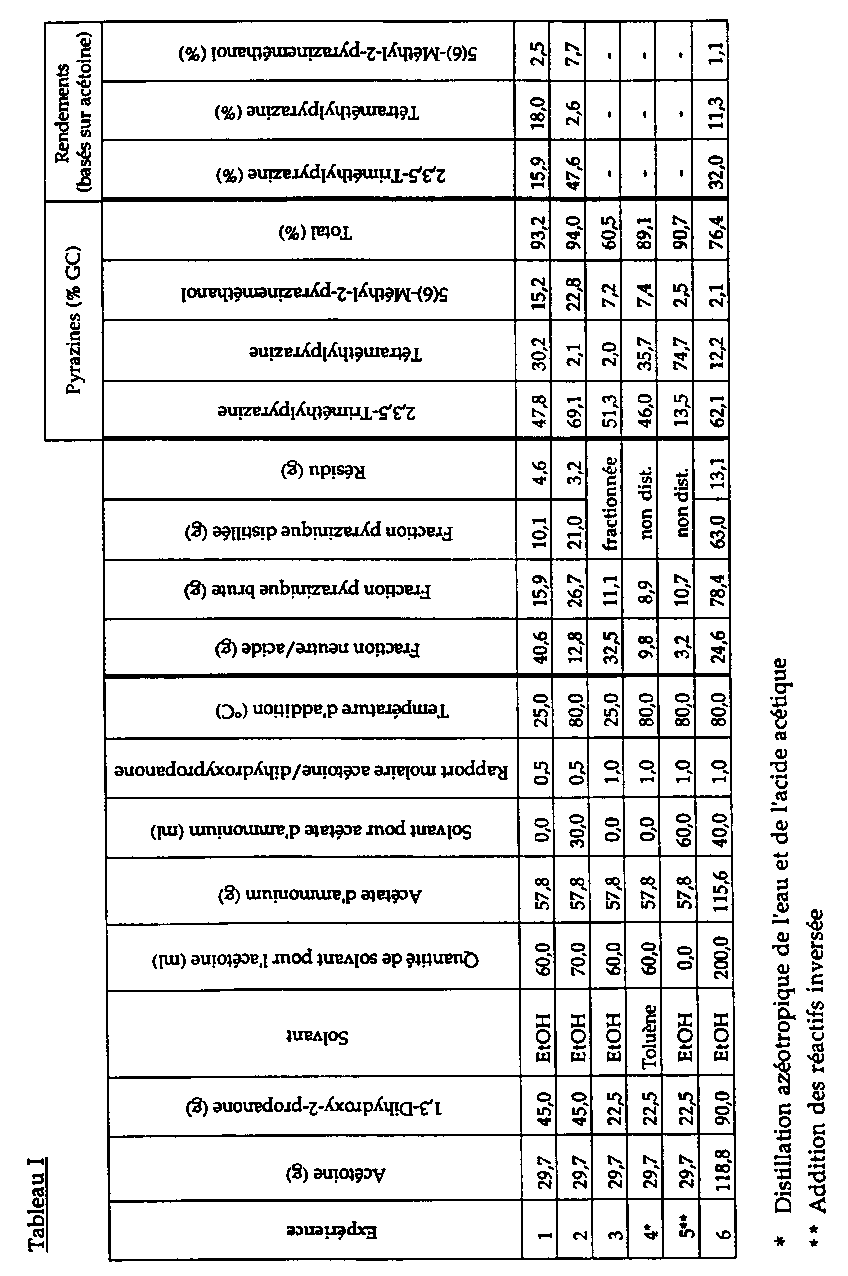

### Exemple 2

### Réaction de 1,3-dihydroxy-2-propanone avec des acyloines variées et acétate ou formiate d'ammonium

On a fait réagir la 1,3-dihydroxy-2-propanone avec une série d'acyloines et acétate ou formiate d'ammonium, en suivant la méthode générale décrite à l'Exemple 1. Les pyrazines obtenues sont indiquées au Tableau II. Ce tableau inclut aussi les résultats de la réaction de l'acétoine avec la (+)-(S)-erythrulose hydratée (réaction 7), utilisée à la place de la 1,3-dihydroxy-2-propanone.

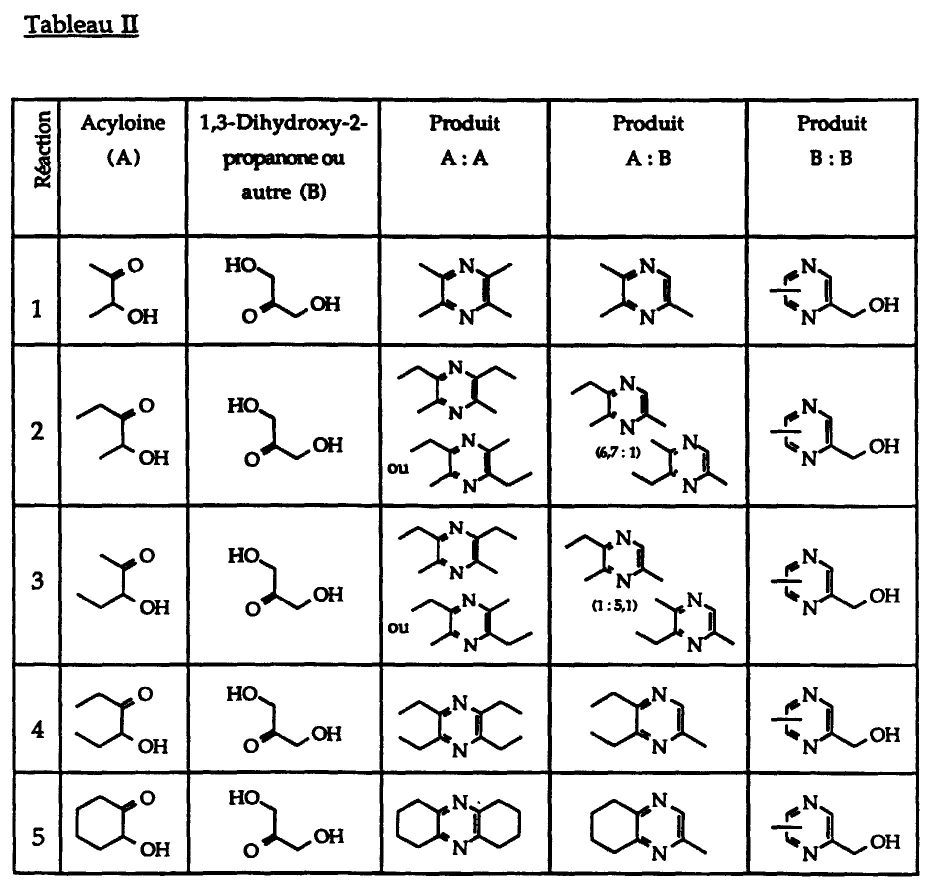

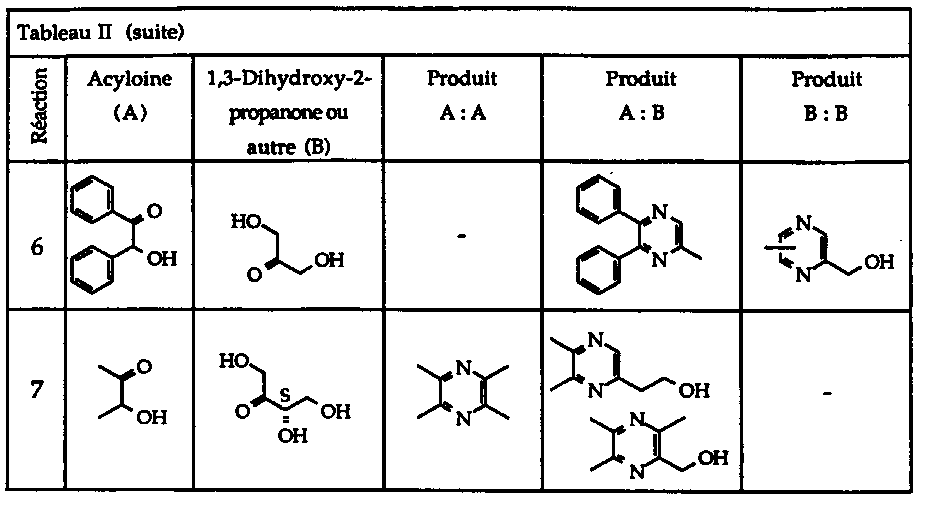

Les conditions des réactions 1 à 6 sont résumées au Tableau III. Les pourcentages de pyrazines indiqués correspondent au contenu de la fraction basique, distillée ou pas. L'identité de tous les produits obtenus, cités au Tableau II ou III, a été confirmée par analyse RMN (¹H et ¹³C) et GC-MS.

**Tableau III**

| Réaction | Acyloine (mole) | 1,3-Dihydroxy -2-propanone (mole) | Acétate ou formiate d'ammonium (mole) | Produit A:A (%) | Produit A:B | |
|---|---|---|---|---|---|---|
| | | | | | (%) | (rend.) |
| 1* | 0,25 | 0,25 | 0,75^{a)} | 20,3 | 72,0 | 22,6 |
| 2 | 0,02 | 0,019 | 0,049 | 17,2 | 86,4 | 42,1 |
| 3 | 0,02 | 0,019 | 0,049 | 12,2 | 86,4 | 41,2 |
| 4* | 0,017 | 0,017 | 0,043 | 18,0 | 59,0 | 25,0 |
| 5 | 0,022 | 0,022 | 0,055 | 18,7 | 79,2 | 48,9 |
| 6** | 0,05 | 0,05 | 0,125 | - | 88,6 | 3,2 |
| 7 | 0,021 | 0,021 ^{b)} | 0,052 | b) | b) | |

| | | | | | | |
|---|---|---|---|---|---|---|
| * Fraction basique n'a pas été distillée | | | | | | |
| ** Solvant utilisé : éthanol/acétate d'éthyle 1:1 | | | | | | |
| a) Formiate d'ammonium | | | | | | |
| b) On a utilisé la (+)-S-érythrulose hydratée à la place de la 1,3-dihydroxy-2-propanone; la distillation et purification de la fraction basique ont fourni une fraction contenant essentiellement le produit A:A (92%), une fraction contenant essentiellement 3,5,6-triméthyl-2-pyrazineméthanol (66%) et une fraction contenant essentiellement 5,6-diméthyl-2-pyrazineéthanol (80,3%) | | | | | | |

## Revendications

1. Procédé pour la préparation d'une pyrazine substituée, de formule 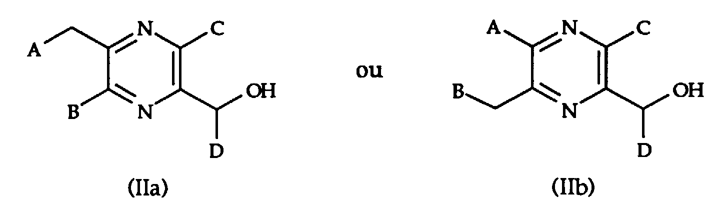 dans laquelle A = B = C = D = R¹ = R², ou dans laquelle A = C = R¹, B = D = R², et R¹ ≠ R², ou A = D = R¹, B = C = R² et R¹ ≠ R², les symboles R¹ et R² représentant chacun un atome d'hydrogène, un radical hydrocarbure saturé ou insaturé, linéaire ou ramifié, ayant de 1 à 6 atomes de carbone ou un groupe -CH₂OH;
ou respectivement de formule 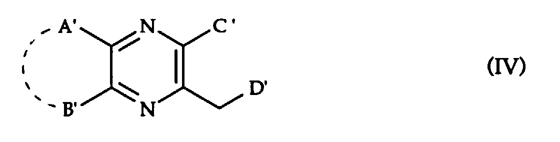 dans laquelle A' = R³, B' = R⁴ ou vice versa et C' = R¹, D' = R², R¹ et R² étant identiques ou différents et ayant le sens indiqué ci-dessus et R³ et R⁴ étant identiques ou différents et représentant chacun un radical hydrocarbure de C₁ à C₆, sauré ou insaturé, linéaire ou ramifié, ou un radical cyclique saturé ou insaturé, ayant 5 ou 6 atomes de carbone dans le cycle, ou étant identiques et représentant un groupe CH ou CH₂ faisant partie d'un cycle tel que représenté par la ligne pointillée, contenant 5 ou 6 atomes de carbone, le procédé étant caractérisé en ce qu'on fait réagir dans un solvant organique inerte un composé de formule 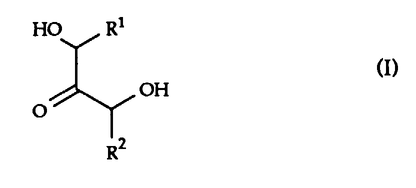 dans laquelle R¹ et R² sont identiques ou différents et ont le sens indiqué ci-dessus, avec un sel d'ammonium, ou respectivement avec un sel d'ammonium et une acyloine de formule 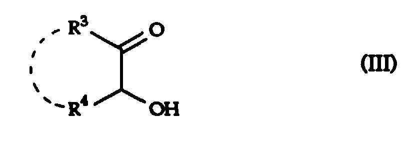 dans laquelle R³ et R⁴ ont le sens indiqué à la formule (IV), et, le cas échéant, on sépare ladite pyrazine du produit de la réaction.

2. Procédé selon la revendication 1, caractérisé en ce que le sel acide d'ammonium est choisi dans le groupe constitué par l'acétate d'ammonium, le citrate d'ammonium, le tartrate d'ammonium, le formiate d'ammonium, l'oxalate d'ammonium, le succinate d'ammonium, le lactate d'ammonium, le sulphamate d'ammonium, le chlorure d'ammonium, le sulphate d'ammonium et le phosphate (II) ou (III) d'ammonium.

3. Procédé selon la revendication 2, caractérisé en ce qu'on utilise l'acétate ou le formiate d'ammonium.

4. Procédé selon l'une des revendications précédentes, caractérisé en ce que le solvant est le méthanol, l'éthanol, le propanol, l'isopropanol, le toluène ou l'acétate d'éthyle, ou encore un mélange d'un ou plusieurs de ces solvants.

5. Procédé selon la revendication 4, caractérisé en ce qu'on utilise en tant que solvant l'éthanol anhydre.

6. Procédé selon l'une des revendications précédentes, caractérisé en ce que la réaction est effectuée à une température comprise entre la température ambiante et la température de reflux du solvant

7. Procédé selon la revendication 6, caractérisé en ce que la réaction est effectuée à la température de reflux du solvant.

8. Procédé selon la revendication 1, caractérisé en ce que l'on ajoute lentement une solution dans l'éthanol de dihydroxycétone (I), ou de dihydroxycétone (I) et d'acyloine (III), au sel d'ammonium, à la température de reflux de l'éthanol.

9. Procédé selon la revendication 8, caractérisé en ce que le sel d'ammonium est l'acétate d'ammonium.

## Claims

1. Process for the preparation of a substituted pyrazine of formula 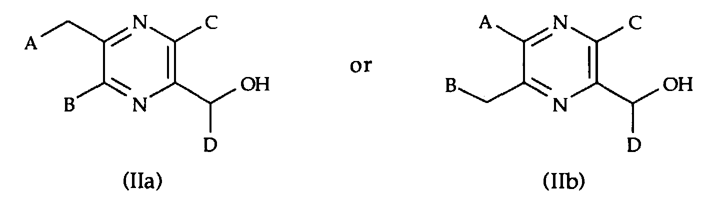 wherein A = B = C = D = R¹ = R², or wherein A = C = R¹, B = D = R², and R¹ ≠ R², or A = D = R¹, B = C = R² and R¹ ≠ R², symbols R¹ and R² representing each a hydrogen atom, a saturated or unsaturated, linear or branched, hydrocarbon radical having 1 to 6 carbon atoms or a -CH₂OH group ;
or respectively of formula 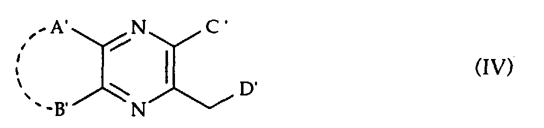 wherein A' = R³, B' = R⁴ or vice versa and C' = R¹, D' = R², R¹ and R² being identical or different and having the above-mentioned meaning and R³ and R⁴ being identical or different and representing each a C₁ to C₆, saturated or unsaturated, linear or branched, hydrocarbon radical, or a saturated or unsaturated cyclic radical, having 5 or 6 carbon atoms in the ring, or being identical and representing a CH or CH₂ group belonging to a ring such as indicated by the dotted line, containing 5 or 6 carbon atoms, the process being characterized by the reaction, in an inert organic solvent, of a compound of formula 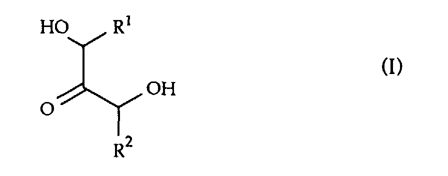 wherein R¹ and R² are identical or different and have the meaning indicated above, with an ammonium salt, or respectively with an ammonium salt and an acyloin of formula 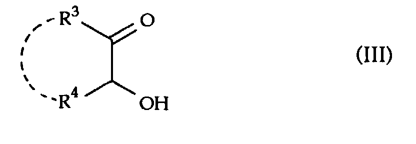 wherein R³ and R⁴ have the meaning indicated in formula (IV) and, if need be, by the separation of said pyrazine from the reaction product.

2. Process according to claim 1, characterized in that the acidic ammonium salt is selected from the group consisting of ammonium acetate, ammonium citrate, ammonium tartrate, ammonium formate, ammonium oxalate, ammonium succinate, ammonium lactate, ammonium sulphamate, ammonium chloride, ammonium sulphate and ammonium phosphate (II) or (III).

3. Process according to claim 2, characterized in that ammonium acetate or formate is used.

4. Process according to anyone of the preceding claims, characterized in that the solvent is methanol, ethanol, propanol, isopropanol, toluene or ethyl acetate, or yet a mixture of two or several of these solvents.

5. Process according to claim 4, characterized in that anhydrous ethanol is used as the solvent.

6. Process according to anyone of the preceding claims, characterized in that the reaction is carried out at a temperature comprised between room temperature and the solvent's reflux temperature.

7. Process according to claim 6, characterized in that the reaction is carried out at the solvent's reflux temperature.

8. Process according to claim 1, characterized in that a solution of dihydroxyketone (I), or of dihydroxyketone (I) and acyloin (III), in ethanol is slowly added to the ammonium salt at the reflux temperature of ethanol.

9. Process according to claim 8, characterized in that the ammonium salt is ammonium acetate.

## Patentansprüche

1. Verfahren zur Herstellung eines substituierten Pyrazins mit der Formel 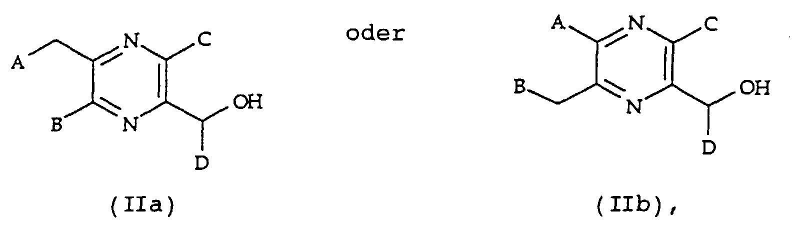 in der A = B = C = D = R¹ = R², oder in der A = C = R¹, B = D = R² und R¹ ≠ R², oder A = D = R¹, B = C = R² und R¹ ≠ R², wobei die Symbole R¹ und R² jeweils für ein Wasserstoffatom, einen gesättigten oder ungesättigten, linearen oder verzweigten Kohlenwasserstoffrest mit von 1 bis 6 Kohlenstoffatomen oder eine Gruppe -CH₂OH stehen;
beziehungsweise mit der Formel 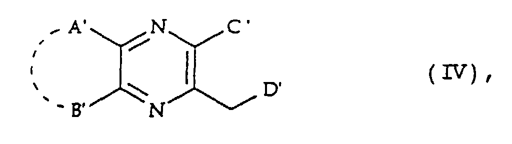 in der A' = R³, B' = R⁴ oder umgekehrt, und C' = R¹, D' = R², R¹ und R² identisch oder verschieden sind und die oben angegebene Bedeutung haben, und R³ und R⁴ identisch oder verschieden sind und jeweils für einen gesättigten oder ungesättigten, linearen oder verzweigten C₁-C₆-Kohlenwasserstoffrest oder einen gesättigten oder ungesättigten cyclischen Rest mit 5 oder 6 Kohlenstoffatomen im Ring stehen, oder identisch sind und für eine Gruppe CH oder CH₂ stehen, die gemäß der Darstellung durch die gestrichelte Linie Teil eines Ringes bildet, der 5 oder 6 Kohlenstoffatome enthält, wobei das Verfahren dadurch gekennzeichnet ist, daß in einem inerten organischen Lösungsmittel eine Verbindung mit der Formel 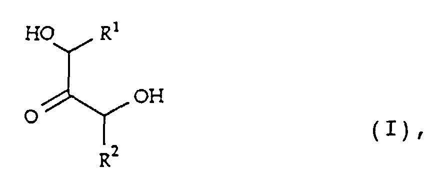 in der R¹ und R² identisch oder verschieden sind und die oben angegebene Bedeutung haben, mit einem Ammoniumsalz bzw. mit einem Ammoniumsalz und einem Acyloin mit der Formel 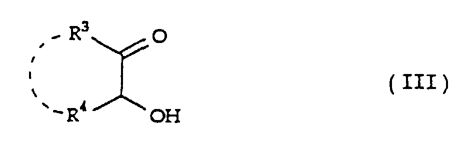 umgesetzt wird, in der R³ und R⁴ die in der Formel (IV) angegebene Bedeutung haben, und das Pyrazin gegebenenfalls vom Reaktionsprodukt getrennt wird.

2. Verfahren nach Anspruch 1, dadurch gekennzeichnet, daß das Ammoniumsalz einer Säure aus der Gruppe ausgewählt wird, die aus Ammoniumacetat, Ammoniumcitrat, Ammoniumtartrat, Ammoniumformiat, Ammoniumoxalat, Ammoniumsuccinat, Ammoniumlactat, Ammoniumsulfamat, Ammoniumchlorid, Ammoniumsulfat, Ammoniumphosphat(II) oder Ammoniumphosphat(III) besteht.

3. Verfahren nach Anspruch 2, dadurch gekennzeichnet, daß Ammoniumacetat oder -formiat verwendet wird.

4. Verfahren nach einem der vorhergehenden Ansprüche, dadurch gekennzeichnet, daß das Lösungsmittel Methanol, Ethanol, Propanol, Isopropanol, Toluol oder Essigsäureethylester oder auch eine Mischung von einem oder mehreren dieser Lösungsmittel ist.

5. Verfahren nach Anspruch 4, dadurch gekennzeichnet, daß als Lösungsmittel wasserfreies Ethanol verwendet wird.

6. Verfahren nach einem der vorhergehenden Ansprüche, dadurch gekennzeichnet, daß die Reaktion bei einer Temperatur durchgeführt wird, die zwischen der Umgebungstemperatur und der Rückflußtemperatur des Lösungsmittels liegt.

7. Verfahren nach Anspruch 6, dadurch gekennzeichnet, daß die Reaktion bei der Rückflußtemperatur des Lösungsmittels durchgeführt wird.

8. Verfahren nach Anspruch 1, dadurch gekennzeichnet, daß eine ethanolische Lösung von Dihydroxyketon (I) bzw. von Dihydroxyketon (I) und Acyloin (III) dem Ammoniumsalz bei der Rückflußtemperatur von Ethanol langsam zugegeben wird.

9. Verfahren nach Anspruch 8, dadurch gekennzeichnet, daß es sich bei dein Ammoniumsalz um Ammoniumacetat handelt.
